Europäisches Patentamt

European Patent Office

Office européen des brevets

(19)

(11) Veröffentlichungsnummer: **0 002 038**
**B1**

(12) **EUROPÄISCHE PATENTSCHRIFT**

(21) Anmeldenummer: **78101343.8**

(22) Anmeldetag: **10.11.78**

(51) Int. Cl.³: **A 61 B 5/14, B 01 L 3/02**

(54) Kapillargefäss

(30) Priorität: **18.11.77 DE 2751503**

(43) Veröffentlichungstag der Anmeldung:
**30.05.79 Patentblatt 79/11**

(45) Bekanntmachung des Hinweises auf die Patenterteilung:
**07.01.81 Patentblatt 81/01**

(84) Benannte Vertragsstaaten:
**BE CH FR GB LU NL SE**

(56) Entgegenhaltungen:
**CH - A - 522 395**
**DE - A - 2 439 218**
**DE - A - 2 558 311**
**FR - A - 2 006 449**
**US - A - 3 141 336**
**US - A - 3 926 521**

(73) Patentinhaber: **Walter Sarstedt Kunstoff-Spritzgusswerk**
**D - 5223 Nümbrecht**
**Rommelsdorf (DE)**

(72) Erfinder: **Sarstedt, Walter**
**D - 5223 Nümbrecht**
**Rommelsdorf (DE)**

(74) Vertreter: **Dahlke, Werner, Dipl.-Ing., et al**
**Frankenforster Strasse 137**
**D - 5060 Bergisch Gladbach 3 (DE)**

Courier Press, Leamington Spa, England.

Kapillargefäß

Die Erfindung bezieht sich auf ein Kapillargefäß zur Entnahme und Aufbewahrung von Blut.

Gefäße zur Aufbewahrung von Blut, insbesondere in Gestalt kleiner, einseitig geschlossener Röhrchen aus Glas oder einem vorzugsweise durchsichtigen Kunststoff sind bekannt und dienen zur Aufbewahrung des mit einer besonderen Blutentnnahmevorrichtung entnommenen Blutes.

Es ist ferner bekannt, kleinere Blutmengen dadurch zu gewinnen, daß man eine Fingerkuppe oder ein Ohrläppchen des Patienten einsticht und das austretende Blut mit einer Kapillare aus Glas oder Kunststoff aufsaugt, die mit ihrem einen Ende in das austretende Blut eingetaucht wird.

Man hat auch schon eine solche enge Glaskapillare in die Bohrung eines Stopfens für ein Aufnahmegefäß gegeben, wobei der Stopfen mit einer Entlüftungsöffnung versehen war.

Ein solches Kapillargefäß zur Entnahme und Aufbewahrung von Blut ist durch die DE—A—25 58 311 bekannt geworden und unfaßt ein Aufnahmegefäß mit einem sich nach unten verjüngenden Unterteil, einem zylindrischen Oberteil und einem sich daran anschließenden Müdungsteil sowie einen in diesen eingesetzten Einsatz, der mit einer einstückig mit ihm ausgestalteten Kapillare zum Ansaugen des Blutes ausgestattet ist. Dabei befindet sich eine Entlüftungsöffnung im Bereich des Einsatzes.

Die vorgenannten bekannten Vorrichtungen haben den Nachteil, daß eine beträchtliche Blutmenge in der Kapillare zurückbleibt, die für die Untersuchung verlorengeht, falls man die nicht besonders ausbläst. Das Ausblasen ist aber nur schwierig unter sterilen Bedingungen vorzunehmen und erfordert einen weiteren Arbeitsgang.

Es ist ferner bekannt, eine solche Kapillare an ihrem hinteren Ende zu erweitern und so an die Entnahmestelle anzusetzen, daß das von der Spitze durch Kapillarwirkung angesaugte Blut dann in den hinteren, erweiterten Teil einfließt. Mit einer solchen trichterförmig erweiterten Kapillare ist es möglich, etwas größere Blutmengen zu entnehmen, als es mit den durchgehend zylindrischen Kapillaren möglich war.

Bei diesen bekannten Kapillargefäßen befand sich zwischen der Spitze mit kapillarer Bohrung und dem hinteren, erweiterten Teil ein engerer Zwischenteil, in dem das Blut infolge der Kapillarwirkung als geschlossene Säule zusammenhing und sich nicht von der Wand löste. Das hatte den Nachteil, daß das von der kapillaren Spitze angesaugte Blut nicht schnell abfließen konnte.

Der Erfindung liegt die Aufgabe zugrunde, ein nach dem vorgenannten Prinzip arbeitendes Kapillargefäß zu schaffen, das eine einfache und saubere Entnahme auch größerer Mengen von aus einer Einstichstelle austretendem Blut durch Kapillarwirkung und eine Aufbewahrung dieses Blutes in einem praktisch geschlossenen Gefäß ermöglicht, das dann anschließend zur weiteren Aufbewahrung bzw. zum Versand völlig dicht verschlossen werden kann.

Diese Aufgabe wird durch die Lehre des Aspruches 1 gelöst.

In Weiterbildung der Erfindung wird vorgeschlagen, daß die Spitze des trichterförmigen Teiles eine Länge von 1 bis 3 mm, vorzugsweise 2 mm, aufweist. Eine solche Bemessung einer zur Blutentnahme dienenden Kapillare ist an sich schon durch die DE—A—24 39 218 bekannt geworden. Dort wurde vorgeschlagen, dem als Kapillare ausgebildeten Mundstück eine Länge von etwa 2 bis 5 mm zu geben.

Weiterbildungen der Erfindung ergeben sich aus den nachfolgenden Unteransprüchen 3 bis 9.

Die Erfindung wird nachstehend in Ausführungsbeispielen anhand der Zeichnung näher erläutert. Dabei zeigen

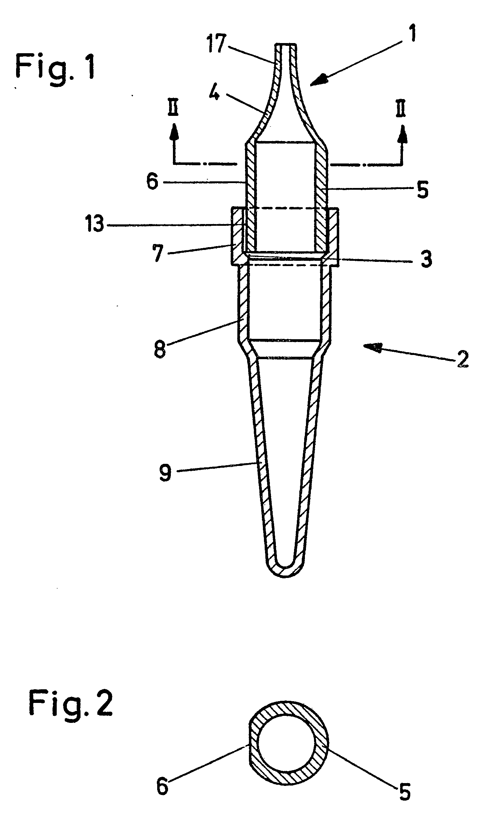

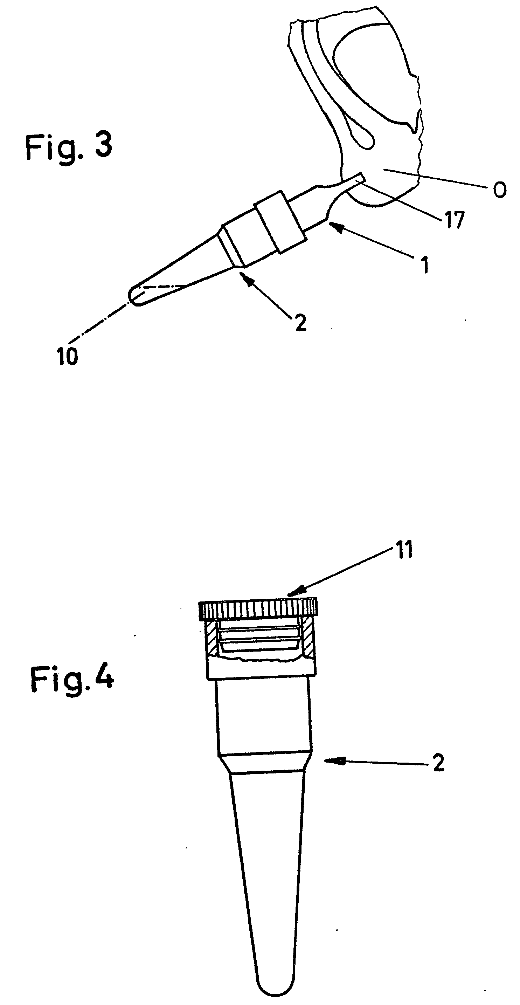

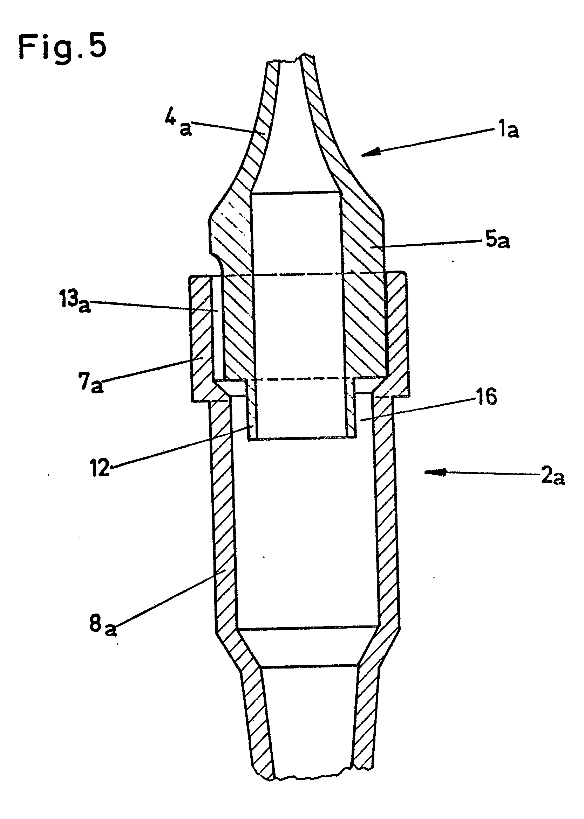

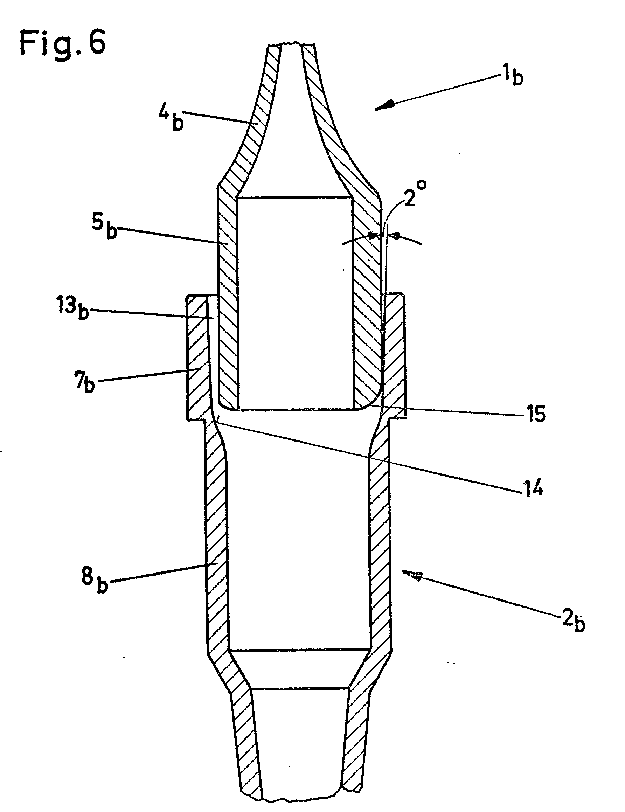

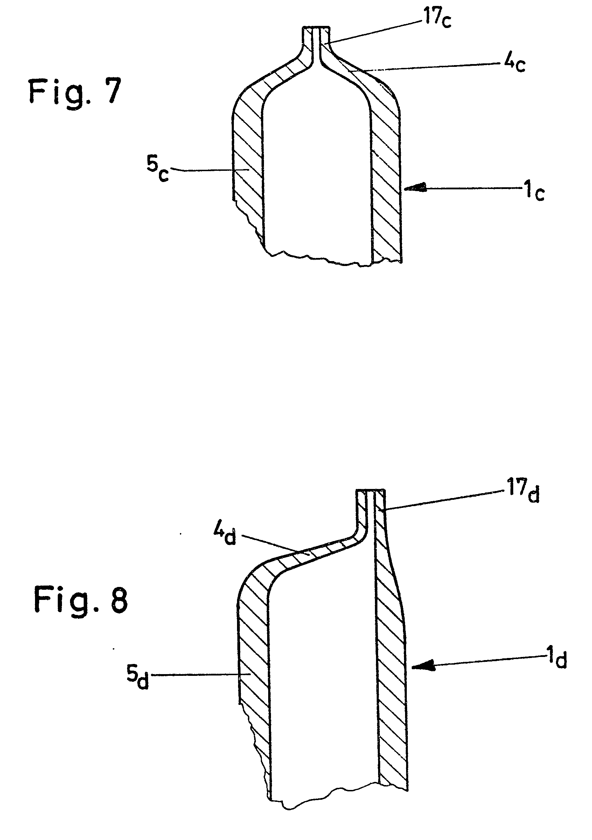

Figur 1 einen vergrößerten Schnitt durch ein erfindungsgemäßes Kapillargefäß, Figur 2 einen Schnitt längs der Linie II—II in Figur 1, Figur 3 eine Ansicht im natürlichen Maßstab eines an das Ohrläppchen eines Patienten angesetzten erfindungsgemäßen Kapillargefäßes, Figur 4 eine teilweise aufgebrochene Ansicht des Aufnahmegefäßes des in den Figuren 1 und 2 dargestellten Kapillargefäßes, verschlossen durch einen Stopfen, Figur 5 einen stärker vergrößerten Teilschnitt durch eine abgewandelte Ausführungsform, Figur 6 einen ebenfalls stärker vergrößerten Teilschnitt durch eine weitere Ausführungsform der Erfindung, Figur 7 einen Teilschnitt, in gleichem Maßstab wie die Darstellung in den Fig. 5 und 6, durch einen abgewandelten Einsatz und Figur 8 einen Teilschnitt im gleichem Maßstab durch einen Einsatz mit exzentrischer Anordnung der Spitze.

Das Kapillargefäß nach den Figuren 1 bis 3 besteht aus einem Aufnahmegefäß 2 und einem eingesteckten Einsatz 1. Der Einsatz ist an seinem oberen Ende trichterförmig (bei 4) verjüngt und mündet in eine sehr kurze kapillare Spitze 27, die, wie in Fig. 3 dargestellt ist, an die Einstichstelle in einem Ohriäppchen 0 (oder auch in einer Fingerkuppe oder dergleichen) angesetz wird. Der Hauptteil 5 des Einsatzes ist, wie aus Fig. 2 ersichtlich ist, im wesentlichen kreiszykindrisch ausgebildet, aber auf einer Seite mit einer Abflachung 6 versehen, die beim Einsetzen des Einsatzes in den Mündungsteil 7 des Aufnahmegefäßes 2 einen Entlüftungskanal 13 zwischen diesen Teilen entstehen läßt, durch den die beim Einlaufen von Blut verdrängte Luft entweichen kann.

Das Aufnahmegefäß ist in seinem Unterteil 9 leicht konisch verjüngt und gestattet somit eine Aufbewahrung und gute Handhabungsmöglichkeit auch kleinster Blutmengen. An diesen konischen Unterteil schließt sich ein kreiszylindrischer Oberteil 8 an, der auch größere Blutmengen aufzubewahren gestattet. Dieser Oberteil geht schließlich in einen erweiterten Mündungsteil 7 über, der den Hauptteil 5 des Einsatzes stramm aufnimmt. Die zylindrische Innenwand dieses Mündungsteiles 7 geht in die engere Innenwand des Oberteils 8 durch eine konische Abschrägung[3] über, auf der sich die Unterkante des Hauptteiles 5 des Einsatzes abstützt.

Wenn die kapillare Spitze 17 des Einsatzes, wie in Fig. 3 dargestellt, an die Einstichstelle eins Ohrläppchens oder einer Fingerkuppe angesetzt wird, wird das austretende Blut von der Kapillare angesaugt. Das Kapillargefäß wird insgesamt etwas schräg gehalten. Das hat zur Folge, daß das zunächst durch Kapillarwirkung in die kapillare Spitze 17 eingesaugte Blut dann unter der Wirkung der Schwerkraft in den erweiterten Teil des Einsatzes einläuft, sich dort von der Wand löst und kontinuierlich weiterfließt. Vom unteren Rand des Einsatzes, der etwas enger gehalten ist, als die Innenwand des Oberteiles des Aufnahmegefäßes, fließt das Blut dann weiter abwärts in das Aufnahmegefäß und sammelt sich am Boden desselben, wie in Fig. 3 bei 10 angedeutet ist. Das Blut fließt solange aus der Einstichstelle in das Aufnahmegefäß, wie die kapillare Spitze 17 in flüssiges Blut eingetaucht bleibt.

Um eine Gerinnung des entnommenen Blutes zu verhindern, kann man in an sich bekannter Weise eine gerinnungshemmende Substanz in das Aufnahmegefäß 2 einbringen.

Nach erfolgter Entnahme der gewünschten Blutmenge wird der Einsatz abgezogen und verworfen und das Aufnahmegefäß durch einen Stopfen 11, wie in Fig. 4 dargestellt ist, verschlossen. Das Blut kann jetzt in diesem Gefäß zentrifugiert oder auch direkt untersucht werden.

Bei der in fig. 5 dargestellten Ausführung ist das Aufnahmegefäß 2a praktisch unverändert, während sich der Hauptteil 5a des Einsatzes 1a mit seinem unteren abgesetzten Ende 12 bis in den Oberteil 8a des Aufnahmegefäßes 2a hineinerstreckt. Die zylindrische Innenwand des Hauptteiles 5a des Einsatzes 1a verläuft glatt und ohne Absatz, so daß das einlaufende Blut gut abfließen kann. Das verjüngte Ende 12 ist außen nur so dick gehalten, daß zwischen ihm und dem umgebenden Oberteil 8a des Aufnahmegefäßes ein so großer Spalt 16 verbleibt, daß er keine Kapillarwirkung besitzt und das Blut ungehindert ablaufen läßt.

Bei der in Fig. 6 dargestellten Ausführungsform ist die Innenwandung des Mündungsteiles 7b des Aufnahmegefäßes 2b leicht konisch zum Öffnungsrand hin erweitert, im Ausführungsbeispiel um 2°. Der Hauptteil 5b des Einsatzes

1b ist, wie bei den vorstehend beschriebenen Ausführungsbeispielen, kreiszylindrisch ausgebildet und an seinem unteren Ende bei 15 abgerundet. Die Abmessungen sind so getroffen, daß sich der Einsatz 1b klemmend in den mit einer konischen Öffnung versehenen Mündungsteil einsetzen läßt.

Da bei dieser Ausbildung kein innerer Absatz an der Stelle des Überganges vom Mündungsteil zum Oberteil des Aufnahmegefäßes zur Abstützung des unteren Randes des Einsatzes mehr benötigt wird, geht hier die leicht konische Innenwand des Mündungsteiles allmählich und ohne scharfe Kanten bei 14 in die zylindrische Innenwand des Oberteiles 8b über. Dadurch wird wiederum ein störungsfreies Abfließen des entnommenen Blutes ermöglicht.

Bei der in Fig. 7 dargestellten Ausführungsform ist die kapillare Spitze 17c des Einsatzes 1c nur ganz kurz gehalten, und zwar so kurz, daß gerade eine kapillare Saugwirkung vorhanden ist. Zwischen dieser kapillaren Spitze 17c und dem Hauptteil 5c des Einsatzes befindet sich ein sehr steiler, trichterförmiger Teil 4c. Diese steile Ausbildung des Trichters gegenüber der schlanken Ausbildung der vorhergehenden Figuren hat den Vorteil, daß das durch die kapillare Spitze angesaugte Blut sofort nach dem Eintritt in den trichterförmigen Teil von der Wand abreißt und unter dem Einfluß der Schwerkraft ungehindert über den untenliegenden Teil der Seitenwand 5c nach unten in das Entnahmegefäß abfließt.

Bei der in Fig. 8 dargestellten Ausführungsform des Einsatzes 1d ist die kapillare Spitze 17d exzentrisch angeordnet und besitzt eine gemeinsame Tangentialebene mit dem ihr benachbarten Teil der Innenwand des Hauptteiles 5d. Bei der Entnahme des Blutes aus der Einstichstelle wird dann der Einsatz so gehalten, daß das durch die kapillare Spitze angesaugte Blut ungehindert abfließen kann. Entsprechend der exzentrischen Lage der kaillaren Spitze 17d ist auch der trichterförmige Teil 4d zwischen dieser Spitze und dem Hauptteil exzentrisch ausgebildet.

Die in den Fig. 1, 5 und 6 dargestellte Abflachung auf einer Seite des Einsatzes ist der Einfachheit halber in den Fig. 7 und 8 für diese Ausbildungen nicht dargestellt bzw. kann unter der Abbruchstele dieser Figuren liegen.

Patentansprüche

1. Kapillargefäß zur Entnahme und Aufbewahrung von Blut, das ein Aufnahmegefäß mit einem sich nach unten verjüngenden Unterteil, einem zylindrischen Oberteil und einem sich daran anschließenden Mündungsteil sowie einen in diesen eingesetzten Einsatz umfaßt, der mit einer einstückig mit ihm ausgestalteten Kapillare zum Ansaugen des Blutes ausgestattet ist, wobei eine Entlüftungsöffnung im Bereich des Einsatzes vorgesehen ist, dadurch gekennzeichnet, daß der Einsatz (1) einen in den Mundungsteil (7) des Aufnahmegefäßes (2) eingesteckten zylindrischen Hauptteil (5) und einen daran anschließenden trichterförmigen Teil (4) unfaßt, dessen Spitze (17) eine Bohrung so geringen Durchmessers aufweist, daß außen befindliches Blut durch Kapillarwirkung eingesaugt wird, während der Hauptteil (5) einen so großen Innendurchmesser hat, daß das eintretende Blut, ohne durch Kapillarwirkung gehemmt zu sein, frei abfließen kann, und daß ferner die Entlüftungsöffnung durch einen Entlüftungskanal (13) zwischen Mündungsteil (7) des Aufnahmegefäßes (2) und Hauptteil (5) des Einsatzes (1) gebildet ist.

2. Kapillargefäß nach Anspruch 1, dadurch gekennzeichnet, daß die Spitze (17) eine Länge von 1 bis 3 mm, vorzugsweise 2 mm, aufweist.

3. Kapillargefäß nach Anspruch 1 und 2, dadurch gekennzeichnet, daß der Entlüftungskanal (13) durch eine Abflachung (6) am äußeren Umfang des Hauptteiles (5) gebildet wird.

4. Kapillargefäß nach Anspruch 1 bis 3, dadurch gekennzeichnet, daß der Innendurchmesser des Hauptteiles (5) des Einsatzes (1) kleiner als der des zylindrischen Oberteils (8) des Aufnahmegefäßes (2) ist.

5. Kapillargefäß nach Anspruch 4, dadurch gekennzeichnet, daß der Hauptteil (5a) des Einsatzes (1a) mit seinem unteren Ende (12) in den Oberteil (8a) des Aufnahmegefäßes (2a) hineinragt.

6. Kapillargefäß nach Anspruch 5, dadurch gekennzeichnet, daß der Außendurchmesser des in den Oberteil (8a) des Aufnahmegefäßes (2a) hineinragenden Endes (12) des Hauptteiles (5a) zur Bildung eines Abstandsspaltes (16) um so viel kleiner als der Innendurchmesser des Oberteiles (8a) ist, daß in ihm keine Kapillarwirkung auftritt.

7. Kapillargefäß nach Anspruch 1 bis 4, dadurch gekennzeichnet, daß sich der Mündungsteil (7b) des Aufnahmegefäßes (2b) zur Öffnung hin leicht konisch, vorzugsweise unter einem Winkel von 1—2°, erweitert und daß der Außendurchmesser des Hauptteiles (5b) des Einsatzes (1b) so bemessen und am unteren Rand so abgerundet ist, daß sich der Einsatz klemmend in den Mündungsteil einsetzen läßt.

8. Kapillargefäß nach Anspruch 7, dadurch gekennzeichnet, daß die leicht konische Innenwand des Mündungsteiles (7b) allmählich und ohne scharfe Kanten (bei 14) in die zylindrische Innenwand des Oberteiles (8b) übergeht.

9. Kapillargefäß nach Anspruch 1 bis 8, dadurch gekennzeichnet, daß der trichterförmige Teil (4d) exzentrisch zum Hauptteil (5d) ausgebildet ist und die kapillare Bohrung in der Spitze (17d) am äußeren Rand der Bohrung des Hauptteiles (5d) liegt.

**Revendications**

1. Récipient à capillaire pour le prélèvement et la conservation de sang, comprenant un récipient collecteur avec une partie inférieure qui s'amincit vers le bas, une partie supérieure cylindrique et une partie d'embouchure qui s'y raccorde, ainsi qu'une pièce rapportée qui est insérée dans cette dernière partie et qui est équipée d'un capillaire formé d'un seul tenant avec elle pour l'aspiration du sang, un orifice d'échappement d'air étant prévu au niveau de la pièce rapportée, caractérisé en ce que la pièce rapportée (1) comprend une partie principale cylindrique (5) insérée dans la partie d'embouchure (7) du récipient collecteur (2) et une partie en entonnoir (4) qui lui fait suite et dont la pointe (17) présente une forure de diamètre suffisamment petit pour que du sang se trouvant à l'extérieur soit aspiré par capillarité, tandis que la partie principale (5) a un diamètre intérieur suffisamment grand pour que le sang qui entre puisse s'écouler librement sans être freiné par effet de capillarité, et en ce qu'en outre l'orifice d'échappement d'air est constitué par un passage d'échappement d'air (13) entre la partie d'embouchure (7) du récipient collecteur (2) et la partie principale (5) de la pièce rapportée (1).

2. Récipient à capillaire selon la revendication 1, caracterisé en se que la pointe (17) a une longueur de 1 à 3 mm, de préférence de 2 mm.

3. Récipient à capillaire selon la revendication 1 ou 2, caracterisé en ce que le passage d'échappement d'air (13) est formé par un méplat (6) sur le pourtour extérieur de la partie principale (5).

4. Récipient à capillaire selon l'une quelconque des revendications 1 à 3, caracterisé en se que le diamètre intérieur de la partie principale (5) de la pièce rapportée (1), est plus petit que celui de la partie supérieure cylindrique (8) du récipient collecteur (2).

5. Récipient à capillaire selon la revendication 4, caracterisé en se que la partie principale (5a) de la pièce rapportée (1a) fait saillie, par son extrémité inférieure (12), dans la partie supérieure (8a) du récipient collecteur (2a).

6. Récipient à capillaire selon la revendication 5, caracterisé en se que le diamètre extérieur de l'extrémité (12) de la partie principale (5a) qui fait saillie dans la partie supérieure (8a) du récipient collecteur (2a) est inférieur au diamètre intérieur de la partie supérieure (8a), de manière à former une fente d'espacement (16), la différence entre ces diamètres étant suffisamment grande pour qu'il ne se produise pas d'effet de capillarité dans la fente.

7. Récipient à capillaire selon l'une quelconque des revendications 1 à 4, caracterisé en se que la partie d'embouchure (7b) du récipient collecteur (2b) s'élargit légèrement en cône vers l'ouverture, de préférence selon un angle de 1 à 2°, et en ce que le diamètre extérieur de la partie principale (5b) de la

pièce rapportée (1b) est dimensionné et son bord inférieur est arrondi de telle sorte que la pièce rapportée puisse être insérée dans la partie d'embouchure en y adhérant.

8. Récipient à capillaire selon la revendication 7, caracterisé en se que la paroi intérieure légèrement conique de la partie d'embouchure (7d) se fond progressivement et sans arrêta vive (en 14) avec la paroi intérieure cylindrique de la partie supérieure (8b).

9. Récipient à capillaire selon l'une quelconque des revendications 1 à 8, caractérisé en se que la partie en entonnoir (4d) est formée en position excentrée par rapport à la partie principale (5d) et la forure capillaire dans la pointe (17d) est située sur le bord extérieur de la cavité de la partie principale (5d).

## Claims

1. Capillary vessel for taking and storing blood, comprising a receptacle with a downward-tapering lower part, a cylindrical upper part and a mouth part adjacent thereto, as well as a fitment which is inserted into the latter and which is provided with a capillary, shaped integrally with it, for drawing the blood in, a vent orifice being provided in the region of the fitment, characterised in that the fitment (1) comprises a cylindrical main part (5), inserted into the mouth part (7) of the receptacle (2), and an adjoining funnel-shaped part (4); the tip (17) of which has a bore of such small diameter that blood present on the outside is drawn in by capillary action, whilst the main part (5) has such a large internal diameter that the blood which enters can flow off freely without being hindered by capillary action, and that additionally the vent orifice is formed by a vent channel (13) between the mouth part (7) of the receptacle (2) and the main part (5) of the fitment (1).

2. Capillary vessel according to Claim 1, characterised in that the tip (17) has a length of 1 to 3 mm, preferably 2 mm.

3. Capillary vessel according to Claim 1 and 2, characterised in that the vent channel (13) is formed by a flattening (6) on the outer periphery of the main part (5).

4. Capillary vessel according to Claim 1 to 3, characterised in that the internal diameter of the main part (5) of the fitment (1) is smaller than that of the cylindrical upper part (8) of the receptacle (2).

5. Capillary vessel according to Claim 4, characterised in that the lower end (12) of the main part (5a) of the fitment (1a) protrudes into the upper part (8a) of the receptacle (2a).

6. Capillary vessel according to Claim 5, characterised in that, to form a spacing gap (16), the external diameter of that end (12) of the main part (5a) which protrudes into the upper part (8a) of the receptacle (2a) is so much smaller than the internal diameter of the upper part (8a) that no capillary action arises therein.

7. Capillary vessel according to Claim 1 to 4, characterised in that the mouth part (7b) of the receptacle (2b) widens slightly conically, preferably under an angle of 1—2°, towards the opening and that the external diameter of the main part (5b) of the fitment (1b) is such, and is rounded at the lower edge in such a way, that the fitment will wedge on insertion into the mouth part.

8. Capillary vessel according to Claim 7, characterised in that the slightly conical inner wall of the mouth part (7b) merges gradually and without sharp edges (at 14) into the cylindrical inner wall of the upper part (8b).

9. Capillary vessel according to Claim 1 to 8, characterised in that the funnel-shaped part (14) is formed eccentrically relative to the main part (5d) and the capillary bore in the tip (17d) is located at the outer edge of the bore of the main part (5d).

Fig. 1

Fig. 2

0 002 038

Fig. 3

O

17

1

2

10

11

Fig. 4

2

2

Fig.5

Fig. 6

Fig. 7

Fig. 8